# EUROPEAN PATENT SPECIFICATION

(11) **EP 0 454 622 B1**
(45) Date of publication and mention of the grant of the patent: **13.10.1999**
(21) Application number: 91810286.4
(22) Date of filing: 16.04.1991
(51) Int. Cl.: C08K 5/32, C10M 133/30, C07C 239/10, C07C 291/04

(54) **Alkenyl substituted stabilizers**
Alkenyl-substituierte Stabilisatoren
Stabilisateurs à substitution alkényle

(30) Priority: 24.04.1990 US 515024; 24.04.1990 US 515013
(43) Date of publication of application: 30.10.1991
(73) Proprietor: Ciba Specialty Chemicals Holding Inc., 4057 Basel (CH)
(72) Inventor: Odorisio, Paul A., Edgewater, N.J. 07020 (US); Babiarz, Joseph E., Amawalk, N.Y. 10501 (US); Meuwly, Roger, Dr., CH-1762 Givisiez (CH); Rutsch, Werner, Dr., CH-1700 Fribourg (CH)

(56) References cited:
- EP-A- 0 246 189
- EP-A- 0 323 409
- FR-A- 1 558 080
- US-A- 4 696 964

## Description

This invention pertains to alkenyl substituted stabilizers, in particular O-alkenyl substituted hydroxylamine compounds and N-alkenyl substituted amine-N-oxide compounds, found to be very effective process stabilizers for polymeric systems, especially those processed at elevated temperatures, as well as for lubricant compositions.

Organic polymeric materials such as plastics and resins are subject to thermal, oxidative and/or photodegradation. A great variety of stabilizers are known in the art for stabilizing a diversity of substrates. Their effectiveness varies depending upon the causes of degradation and the substrate to be stabilized. In general, it is difficult to predict which stabilizers will be most effective and most economical for any one area of stabilization. For example, stabilizer effectiveness in reducing volatility may depend upon preventing bond scission in the substrate molecule. Limiting embrittlement and retaining elasticity in a polymer or rubber may require prevention of excessive crosslinking and/or chain scission. Prevention of discoloration may require inhibiting reactions which yield new chromophores or color bodies in the substrate or stabilizer. Problems of process stability and incompatibility must also be considered.

Various organic hydroxylamine compounds are generally known and some are commerically available. A number of patents disclose nitrogen-substituted hydroxylamines as antioxidant stabilizers for various substrates including polyolefins, polyesters, polyurethanes, elastomers and poly(arylene sulfides). United States Patent Nos. 3,432,578; 3,644,278; 3,778,464; 3,408,422; 3,926,909; 4,316,996; 4,386,224 and 4,782,105 are representative of such patents which basically disclose N,N-dialkyl-, N,N-diaryl- and N,N-diaralkylhydroxylamines and their stabilization effects.

In addition, various N,N,O-trisubstituted and N,O-disubstituted hydroxylamines are disclosed in the literature. For example, U.S. Patent No. 3,184,500 describes R₂NOCH₂CH₂O-Acyl where R is lower alkyl; U.S. 3,344,190 describes R₂NOCH₂CH₂OH where R is lower alkyl; U.S. 3,869,278 describes (R)₂NOR₁ where R is alkyl and R₁ is inter alia alkyl and phenyl, as fruit abscission agents; German 1,237,129 discloses the preparation of N,N,O-trisubstituted hydroxylamines by UV irradiation of amine oxides; L.A. Paquette [J. Org. Chem. 29, 3545 (1964)] describes the preparation of R₂NCH₂CH₂ONH₂ where R₂N are heterocyclic groups; E.I. Schumann et al. [J. Med. Chem. 7, 329 ( (1964)] describe the preparation of several O-aralkylhydroxylamines useful as pharmaceutical agents; A. T. Fuller [J. Chem. Soc., 1947, 963 (pt 2)] reports the preparation of a series of O-alkyl and O,O'-alkylenehydroxylamines from hydroxyurethanes with potential antibacterial activity; Bernhart [Tetrahedron Letters, 29, 2493 (1974)] describes the preparation of a series of N,O-dialkylhydroxylamines by the reduction of O-alkylbenzaldoximes; B.V. Tronov et al. [Chem. Abst. 66, 37339f (1967)] describe the preparation and properties of N-alkoxydiethylamines; O-alkylation of N,N-diethylhydroxylamine is described by E. Flesia et al. [Tetrahedron Letters, 1979, 197]; I. M. Bortori et al. [Chem. Abst. 73, 55743g (1969)] describe the synthesis and antibacterial properties of N-(aryloxy)diethylamines; F. Klages [Ber., 96, 2387 (1963)] describes the preparation of N,N-dibenzyl-O-ethyl-hydroxylamine and N,N-di-tert-butyl-O-benzylhydroxylamine; and Japanese 69/25772 [Chem. Abst. 72, 78685h(1970)] describes the preparation of (R')(OR)NCH₂CH₂CO(C₆H₄X) useful as anti-inflammatory drugs. It is noted that these various compounds are all indicated for pharmaceutical or agricultural uses.

O-Alkenyl substituted hydroxylamines are also known, but again only for pharmaceutical or agricultural uses. Representative of such references are European 29,171; European 103,895; U.S. 4,226,612; Japanese Sho 57-45143; German 2,541,702; German 2,439,104; U.S. 3,755,577; German 3,728,278 and European 110,280.

U.S. Patent No. 4,626,411 teaches the use of N,N,O-trisubstituted hydroxylamines as corrosion inhibitors. The O-substitution in said compounds is given as hydrogen, lower alkyl or aryl. There is no disclosure or suggestion of O-alkenyl substitution in said compounds.

U.S. Patent No. 4,696,964 does teach the use of N,O-disubstituted and N,N,O-trisubstituted hydroxylamines as stabilizers for a variety of substrates. There is no disclosure or suggestion in this patent that any of the groups being substituted on the hydroxylamine moiety should be alkenyl. The advantages observed with the instant compounds as stabilizers were not even comtemplated by this closest of the prior art.

Saturated tertiary amine N-oxides are known as non-ionic surfactants. The oxidation and rearrangement of selected N-alkenyl(allyl) tertiary amine N-oxides to the corresponding O-alkenyl(allyl) hydroxylamines are taught by A.C. Cope et al., J. Am. Chem. Soc., 71, 3423 (1949); R.F. Kleinschmidt and A.C. Cope, J. Am. Chem. Soc., 66, 1929 (1944); S. Inoue et al., Chem. Letters, 1986, 2035; and Y. Inouye et al., J. Org. Chem. 41, 300 (1976). The use of N-alkenyl-N-oxides of tertiary amines as stabilizers for organic substrates is not taught in the prior art.

The instant invention pertains to stabilized compositions which comprise
(a) a synthetic polymer or a lubricant subject to oxidative, thermal or actinic-induced degradation, and
(b) 0.01 to 5% by weight of the stabilized composition of a compound of formula I 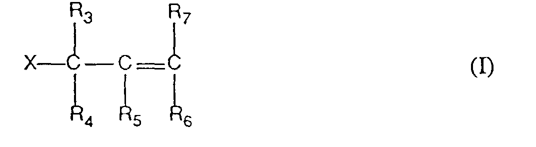
wherein X is a group of formula Ia or Ib, 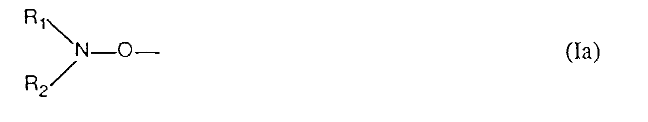 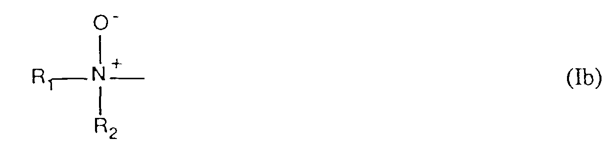
R₁ and R₂ are independently hydrogen, a straight or branched chain alkyl of 1 to 36 carbon atoms, alkyl of 1 to 18 carbon atoms terminated by a group -OR₈, -NR₉R₁₀, -SR₁₁, -COOR₁₂ or -CONR₁₃R₁₄ or interrupted by arylene of 6 to 10 carbon atoms, -O-, -S-, -SO-, -SO₂-, -COO-, -OCO-, -CONR₁₅-, -NR₁₅CO- or -NR₁₆- where R₈, R₉, R₁₀, R₁₁, R₁₂, R₁₃, R₁₄, R₁₅ and R₁₆ are independently hydrogen, alkyl of 1 to 18 carbon atoms or alkenyl of 3 to 6 carbon atoms; or R₁ and R₂ are independently cycloalkyl of 5 to 12 carbon atoms, phenylalkyl of 7 to 15 carbon atoms, said phenylalkyl substituted on the phenyl ring by alkyl of 1 to 18 carbon atoms or by α-cumyl; aryl of 6 to 14 carbon atoms, said aryl substituted by one or two alkyl of 1 to 24 carbon atoms,
R₃ and R₄ are independently hydrogen, alkyl of 1 to 9 carbon atoms, said alkyl substituted by -OH or by acetoxy; alkenyl of 3 to 6 carbon atoms or aryl of 6 to 10 carbon atoms, and
R₅, R₆ and R₇ are independently hydrogen, alkyl of 1 to 9 carbon atoms, said alkyl substituted by -OH or by acetoxy; alkenyl of 3 to 6 carbon atoms, aryl of 6 to 10 carbon atoms, or, when X is a group of formula Ia, a group of formula IIa 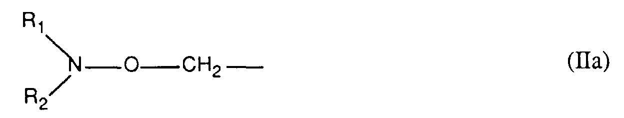
or, when X is a group of formula Ib, a group of formula IIb, 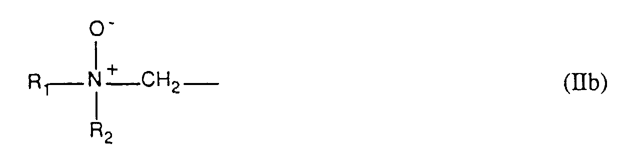 R₃ and R₄, or R₃ and R₅, or R₃ and R₇, or R₄ and R₅, or R₄ and R₇, or R₅ and R₆, or R₆ and R₇ together are straight or branched chain alkylene of 2 to 8 carbon atoms to form a cycloalkyl or cycloalkenyl ring with 5 or 6 ring atoms.

The compounds of this invention possess excellent properties in several important facts. These are:
(1) they exhibit superior polymer stabilization properties during the processing of polymers at elevated temperatures;
(2) they provide resistance to color development during processing especially when used in conjunction with a phenolic antioxidant;
(3) they are less prone to oxidation by ambient air during storage or in handling;
(4) they do not interact with polymerization catalysts such as the sulfonic acid catalysts used during the curing of aminoplast resin systems;
(5) they are more soluble in typical organic solvents and are more compatible with the polymeric substrates being stabilized;
(6) they resist moisture pickup and provide low water carry-over properties; and
(7) they inhibit sludge formation in and control viscosity increase of motor oil formulations during use.

Preferably the instant compounds of formula I are those where
R₁ and R₂ are independently straight or branched chain alkyl of 1 to 20 carbon atoms, alkyl of 1 to 18 carbon atoms terminated by a group -OR₈, -NR₉R₁₀, -SR₁₁, -COOR₁₂ or -CONR₁₃R₁₄ or interrupted by phenylene, -O-, -S-, -SO-, -SO₂-, -COO-, -OCO-, -CONR₁₅-, -NR₁₅CO- or -NR₁₆-, where R₈, R₉, R₁₀, R₁₁, R₁₂, R₁₃, R₁₄, R₁₅ and R₁₆ are independently hydrogen or alkyl of 1 to 18 carbon atoms; or R₁ and R₂ are independently cycloalkyl of 5 to 7 carbon atoms, benzyl, benzyl substituted on the phenyl ring by alkyl of 1 to 12 carbon atoms or by α-cumyl; aryl of 6 to 14 carbon atoms or said aryl substituted by one or two alkyl of 1 to 24 carbon atoms,
R₃ is hydrogen or alkyl of 1 to 4 carbon atoms,
R₄ is hydrogen, alkyl of 1 to 4 carbon atoms or phenyl,
R₅ and R₆ are independently hydrogen, alkyl of 1 to 9 carbon atoms, or, when X is a group of formula Ia, a group of formula IIa or, when X is a group of formula Ib, a group of formula IIb,
R₇ is hydrogen, alkyl of 1 to 9 carbon atoms or phenyl.

Most preferably the instant compounds of formula 1 are those where
R₁ and R₂ are independently alkyl of 8 to 18 carbon atoms, cyclohexyl, benzyl, phenyl, 1-naphthyl, or said phenyl or said naphthyl substituted by one or two alkyl of 4 to 12 carbon atoms,
R₃ is hydrogen,
R₄ is hydrogen or methyl,
R₅ and R₆ are independently hydrogen or, when X is a group of formula Ia, a group of formula IIa or, when X is a group of formula Ib, a group of formula IIb, and
R₇ is hydrogen.

When any of the aforementioned groups are alkyl, they are, for example, methyl, ethyl, isopropyl, n-butyl, sec-butyl, tert-butyl, isoamyl, tert-amyl, n-hexyl,2-ethylhexyl, isooctyl, n-octyl, nonyl, decyl, undecyl, lauryl, tridecyl, tetradecyl, hexadecyl, heptadecyl, octadecyl, eicosyl, tricontyl and branched isomers thereof.

Alkenyl of 3 to 6 carbon atoms is for example allyl or 2-butenyl.

Cycloalkyl of 5 to 12 carbon atoms includes, for example, cyclopentyl, cyclohexyl, cycloheptyl, cyclooctyl and cyclododecyl.

Phenylalkyl of 7 to 15 carbon atoms includes, for example, benzyl, phenethyl, α-methylbenzyl and β-methylphenethyl.

Aryl includes, for example, phenyl, 1-naphthyl, 2-naphthyl, xenyl, anthracyl and phenanthryl.

Aryl substituted by alkyl is, for example, tolyl, xylyl, mesityl, ethylphenyl, tert-butylphenyl, tert-octylphenyl, tert-dodecylphenyl, tert-butylnaphthyl or tert-octylnaphthyl.

Alkylene of 2 to 8 carbon atoms is for example 1,3-trimethylene, 1,4-tetramethylene or 1,5-pentamethylene.

Arylene of 6 to 10 carbon atoms is for example 1,4-phenylene.

The starting materials for making the instant compounds of formula I are largely items of commerce and can be made by known methods.

The instant compounds of formula I wherein X is a group of formula Ib are conveniently prepared by allylation of a secondary amine using an alkenyl (allyl) halide by the method taught by A.W. Weston et al., J. Am. Chem. Soc., 65, 674 (1943) followed by oxidation of the N-alkenyl(allyl) tertiary amine to the corresponding N-alkenyl(allyl) amine-N-oxide.

The instant compounds of formula I wherein X is a group of formula Ia are conveniently prepared by two main routes. One involves the rearrangement of the N-alkenyl(allyl) tertiary amine N-oxide to the corresponding O-alkenyl(allyl) hydroxylamine as taught by A.C. Cope et al., J. Am. Chem. Soc., 71, 3423 (1949); R.F. Kleinschmidt and A.C. Cope, J. Am. Chem. Soc., 66, 1929 (1944); S. Inoue et al., Chem. Letters, 1986, 2035; and Y. Inouye et al., J. Org. Chem., 41, 300 (1976).

The second route is starting from the corresponding hydroxylamine, usually an N,N-disubstituted hydroxylamine, followed by a metathesis reaction between an alkenyl (allyl) halide and the hydroxylamine in the presence of an alkali such as sodium hydride, sodium carbonate or sodamide.

The preparation of a large variety of N,N-disubstituted hydroxylamines is described in United States Patent Nos. 4,590,231; 4,612,393; 4,646,221; 4,663,373; 4,666,962; 4,666,963; 4,668,721; 4,668,727; 4,673,700; 4,696,964; 4,703,013; 4,717,748; 4,720,517; 4,749,733; 4,753,972; 4,757,102; 4,758,614; 4,760,179; 4,782,105; 4,888,444 and 4,898,901, and in European Laid Open Print 273,011.

A preferred embodiment of the instant compounds is derived from hydrogenated tallow amine which is a mixture of secondary amines of the general formula

T₁T₂NH

where the typical distribution of the alkyl substituents is as follows:

| T₁ | T₂ | % |
|---|---|---|
| C₁₆ | C₁₄ | 1.9 |
| C₁₆ | C₁₆ | 12.4 |
| C₁₆ | C₁₇ | 2.8 |
| C₁₆ | C₁₈ | 36.0 |
| C₁₇ | C₁₈ | 3.9 |
| C₁₈ | C₁₈ | 39.0 |
| other | other | 4.0 |

It is clear that the di(hydrogenated tallow)amine originating from animal sources may well vary somethat in the specific distribution of the alkyl substituents, but the di(hydrogenated tallow)amine contains major amounts of N,N-dihexadecylamine, N,N-dioctadecylamine and N-hexadecyl-N-octadecylamine. The individual components of the mixture can be separated by distillation under high vacuum.

However, for the purposes of this invention, there is no need to carry out such a separation and the hydroxylamine prepared from the di(hydrogenated tallow)amine represents a preferred starting material for making the O-alkenyl compounds of this invention.

The compositions where component (a) is a synthetic polymer are especially part of this invention, most particularly when the synthetic polymer is a polyolefin such as polypropylene.

The instant compounds are effective stabilizers for organic materials or compositions of matter comprising organic matter in that they reduce degradation resulting from long term oxidative and/or thermal aging and effectively protect said materials from actinic radiation.

In addition, the instant compounds show little tendency to evaporate or sublime from the organic compositions during thermal processing. Thus, the instant compounds are effective process stabilizers for organic polymers processed at elevated temperatures.

In still another end-use application, the instant compounds of formula I, especially those where R₁ and R₂ are aryl, are useful in stabilizing, by inhibiting oxidation, industrial lubricants such as lubricating oils, turbine oils, transformer oils, transmission fluids, glass-annealing oils, greases, steam turbine oils, gasoline engine oils, diesel engine oils, jet engine oils, metal working fluids and the like. They are also effective in stabilizing waxes, heating oil, bunker and residual oils, asphalt, gasoline and jet engine fuel. They are especially effective in stabilizing lubricant, e.g. motor oil or gasoline engine oil.

Still another aspect of the instant invention are the novel compounds of formula III 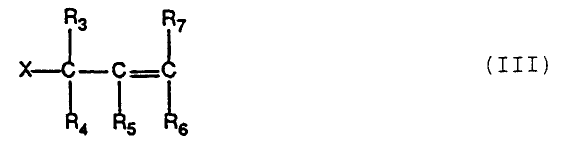 wherein X is a group of formula IIIa or IIIb, 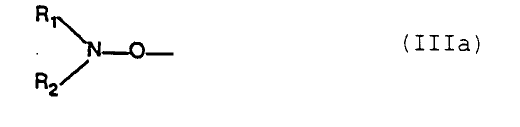 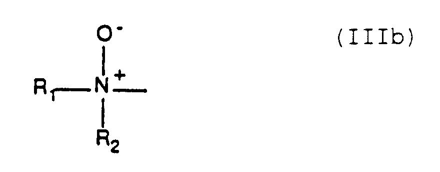
R₁ and R₂ are independently alkyl of 8 to 36 carbon atoms, benzyl, benzyl substituted on the phenyl ring by alkyl of 1 to 18 carbon atoms or by α-cumyl; aryl of 6 to 14 carbon atoms or said aryl substituted by one or two alkyl of 1 to 24 carbon atoms,
R₃ and R₄ are independently hydrogen, alkyl of 1 to 9 carbon atoms, alkenyl of 3 to 6 carbon atoms or aryl of 6 to 10 carbon atoms, and
R₅, R₆ and R₇ are independently hydrogen, alkyl of 1 to 9 carbon atoms, alkenyl of 3 to 6 carbon atoms, aryl of 6 to 10 carbon atoms or, when X is a group of formula IIIa, a group of formula IVa 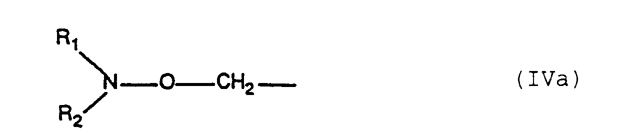 or, when X is a group of formula IIIb, a group of formula IVb,

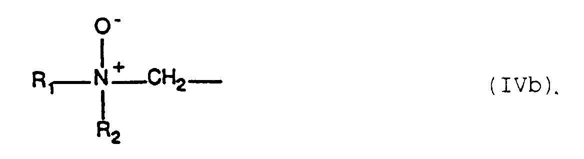

The most preferred novel compounds of the instant invention are those where
R₁ and R₂ are independently alkyl of 8 to 18 carbon atoms, benzyl, phenyl, 1-naphthyl, or said phenyl or said naphthyl substituted by one or two alkyl of 4 to 12 carbon atoms,
R₃ is hydrogen,
R₄ is hydrogen or methyl,
R₅ and R₆ are independently hydrogen or, when X is a group of formula IIIa, a group of formula IVa or, when X is a group of formula IIIb, a group of formula IVb, and
R₇ is hydrogen.

The most preferred novel compounds of this invention are those where
R₁ and R₂ are independently alkyl of 12 to 18 carbon atoms, benzyl, phenyl, 1-naphthyl or said phenyl or said naphthyl substituted by one or two alkyl of 4 to 8 carbon atoms,
R₃ is hydrogen,
R₄ is hydrogen,
R₅ is hydrogen or, when X is a group of formula IIIa, a group of formula IVa or, when X is a group of formula IIIb, a group of formula IVb, and
R₆ is hydrogen or, when X is a group of formula IIIa, a group of formula IVa or, when X is a group of formula IIIb, a group of formula IVb, and
R₇ is hydrogen.

Substrates in which the compounds of this invention are particularly useful are synthetic polymers, e.g. polyolefins such as polyethylene and polypropylene; polystyrene, including especially impact polystyrene; ABS resin; elastomers such as e.g. butadiene rubber, EPM, EPDM, SBR and nitrile rubber.

In general polymers which can be stabilized include
1. Polymers of monoolefins and diolefins, for example polyethylene (which optionally can be crosslinked), polypropylene, polyisobutylene, polybutene-1, polymethylpentene-1, polyisoprene or polybutadiene, as well as polymers of cycloolefins, for instance of cyclopentene or norbornene.
2. Mixtures of the polymers mentioned under 1), for example mixtures of polypropylene with polyisobutylene.
3. Copolymers of monoolefins and diolefins with each other or with other vinyl monomers, such as, for example, ethylene/propylene, propylene/butene-1, propylene/ isobutylene, ethylene/butene-1, propylene/butadiene, isobutylene/isoprene, ethylene/alkyl acrylates, ethylene/ alkyl methacrylates, ethylene/vinyl acetate or ethylene/acrylic acid copolymers and their salts (ionomers) and terpolymers of ethylene with propylene and a diene, such as hexadiene, dicyclopentadiene or ethylidene-norbornene.
4. Polystyrene, poly-(p-methylstyrene).
5. Copolymers of styrene or methylstyrene with dienes or acrylic derivatives, such as, for example, styrene/butadiene, styrene/acrylonitrile, styrene/ethyl methacrylate, styrene/butadiene/ethyl acrylate, styrene/acrylonitrile/methyl acrylate; mixtures of high impact strength from styrene copolymers and another polymer, such as, for example, from a polyacrylate, a diene polymer or an ethylene/propylene/diene terpolymer; and block polymers of styrene, such as, for example, styrene/butadiene/styrene, styrene/isoprene/styrene, styrene/ethylene/butylene/styrene or styrene/ethylene/propylene/styrene.
6. Graft copolymers of styrene, such as, for example, styrene on polybutadiene, styrene and acrylonitrile on polybutadiene, styrene and alkyl acrylates or methacrylates on polybutadiene, styrene and acrylonitrile on ethylene/propylene/diene terpolymers, styrene and acrylonitrile on polyacrylates or polymethacrylates, styrene and acrylonitrile on acrylate/butadiene copolymers, as well as mixtures thereof with the copolymers listed under 5), for instance the copolymer mixtures known as ABS-, MBS-, ASA- or AES-polymers.
7. Halogen-containing polymers, such as polychloroprene, chlorinated rubbers, chlorinated or sulfochlorinated polyethylene, epichlorohydrin homo- and copolymers, polymers from halogen-containing vinyl compounds, as for example, polyvinylchloride, polyvinylidene chloride, polyvinyl fluoride, polyvinylidene fluoride, as well as copolymers thereof, as for example, vinyl chloride/vinylidene chloride, vinyl chloride/vinyl acetate, vinylidene chloride/vinyl acetate copolymers, or vinyl fluoride/vinyl ether copolymers.
8. Polymers which are derived from α,β-unsaturated acids and derivatives thereof, such as polyacrylates and polymethacrylates, polyacrylamide and polyacrylonitrile.
9. Copolymers from the monomers mentioned under 8) with each other or with other unsaturated monomers, such as, for instance, acrylonitrile/butadiene, acrylonitrile/alkyl acrylate, acrylonitrile/alkoxyalkyl acrylate or acrylonitrile/vinyl halogenide copolymers or acrylonitrile/alkyl methacrylate/butadiene terpolymers.
10. Polymers which are derived from unsaturated alcohols and amines, or acyl derivatives thereof or acetals thereof, such as polyvinyl alcohol, polyvinyl acetate, polyvinyl stearate, polyvinyl benzoate, polyvinyl maleate, polyvinyl-butyral, polyallyl phthalate or polyallyl-melamine.
11. Homopolymers and copolymers of cyclic ethers, such as polyalkylene glycols, polyethylene oxide, polypropylene oxide or copolymers thereof with bis-glycidyl ethers.
12. Polyacetals, such as polyoxymethylene and those polyoxymethylenes which contain ethylene oxide as comonomer.
13. Polyphenylene oxides and sulfides, and mixtures of polyphenylene oxides with polystyrene.
14. Polyurethanes which are derived from polyethers, polyesters or polybutadienes with terminal hydroxyl groups on the one side and aliphatic or aromatic polyisocyanates on the other side, as well as precursors thereof (polyisocyanates, polyols or prepolymers).
15. Polyamides and copolyamides which are derived from diamines and dicarboxylic acids and/or from aminocarboxylic acids or the corresponding lactams, such as polyamide 4, polyamide 6, polyamide 6/6, polyamide 6/10, polyamide 11, polyamide 12, poly-2,4,4-trimethylhexamethylene terephthalamide, poly-p-phenylene terephthalamide or poly-m-phenylene isophthalamide, as well as copolymers thereof with polyethers, such as for instance with polyethylene glycol, polypropylene glycol or polytetramethylene glycols.
16. Polyureas, polyimides and polyamide-imides.
17. Polyesters which are derived from dicarboxylic acids and diols and/or from hydroxycarboxylic acids or the corresponding lactones, such as polyethylene terephthalate, polybutylene terephthalate, poly-1,4-dimethylol-cyclohexane terephthalate, poly-[2,2-(4-hydroxyphenyl)-propane] terephthalate and polyhydroxybenzoates as well as block-copolyether-esters derived from polyethers having hydroxyl end groups.
18. Polycarbonates.
19. Polysulfones, polyethersulfones and polyetherketones.
20. Crosslinked polymers which are derived from aldehydes on the one hand and phenols, ureas and melamines on the other hand, such as phenol/formaldehyde resins, urea/formaldehyde resins and melamine/formaldehyde resins.
21. Drying and non-drying alkyd resins.
22. Unsaturated polyester resins which are derived from copolyesters of saturated and unsaturated dicarboxylic acids with polyhydric alcohols and vinyl compounds as crosslinking agents, and also halogen-containing modifications thereof of low flammability.
23. Thermosetting acrylic resins, derived from substituted acrylic esters, such as epoxy-acrylates, urethane-acrylates or polyester acrylates.
24. Alkyd resins, polyester resins or acrylate resins in admixture with melamine resins, urea resins, polyisocyanates or epoxide resins as crosslinking agents.
25. Crosslinked epoxide resins which are derived from polyepoxides, for example from bis-glycidyl ethers or from cycloaliphatic diepoxides.
26. Natural polymers, such as cellulose, rubber, gelatin and derivatives thereof which are chemically modified in a polymer homologous manner, such as cellulose acetates, cellulose propionates and cellulose butyrates, or the cellulose ethers, such as methyl cellulose.
27. Mixtures of polymers as mentioned above, for example PP/EPDM, Polyamide 6/EPDM or ABS, PVC/EVA, PVC/ABS, PVC/MBS, PC/ABS, PBTP/ABS.
28. Naturally occuring and synthetic organic materials which are pure monomeric compounds or mixtures of such compounds, for example mineral oils, animal and vegetable fats, oil and waxes, or oils, fats and waxes based on synthetic esters (e.g. phthalates, adipates, phosphates or trimellitates) and also mixtures of synthetic esters with mineral oils in any weight ratios, which materials may be used as plasticizers for polymers or as textile spinning oils, as well as aqueous emulsions of such materials.
29. Aqueous emulsions of natural or synthetic rubber, e.g. natural latex or latices of carboxylated styrene/butadiene copolymers.
30. Polysiloxanes such as the soft, hydrophilic polysiloxanes described, for example, in U.S. Patent No. 4,259,467; and the hard polyorganosiloxanes described, for example, in U.S. Patent No. 4,355,147.
31. Polyketimines in combination with unsaturated acrylic polyacetoacetate resins or with unsaturated acrylic resins. The unsaturated acrylic resins include the urethane acrylates, polyether acrylates, vinyl or acryl copolymers with pendant unsaturated groups and the acrylated melamines. The polyketimines are prepared from polyamines and ketones in the presence of an acid catalyst.
32. Radiation curable compositions containing ethylenically unsaturated monomers or oligomers and a polyunsaturated aliphatic oligomer.
33. Epoxymelamine resins such as light-stable epoxy resins crosslinked by an epoxy functional coetherified high solids melamine resin such as LSE 4103 (Monsanto).

In general, the compounds of the present invention are employed in from about 0.01 to about 5% by weight of the stabilized composition, although this will vary with the particular substrate and application. An advantageous range is from about 0.05 to about 2%, and especially 0.1 to about 1%.

The stabilizers of the instant invention may readily be incorporated into the organic polymers by conventional techniques, at any convenient stage prior to the manufacture of shaped articles therefrom. For example, the stabilizer may be mixed with the polymer in dry powder form, or a suspension or emulsion of the stabilizer may be mixed with a solution, suspension, or emulsion of the polymer. The resulting stabilized polymer compositions of the invention may optionally also contain various conventional additives, such as the following.

### 1. Antioxidants

### 1.1. Alkylated monophenols, for example,

2,6-di-tert-butyl-4-methylphenol
2-tert.butyl-4,6-dimethylphenol
2,6-di-tert-butyl-4-ethylphenol
2,6-di-tert-butyl-4-n-butylphenol
2,6-di-tert-butyl-4-i-butylphenoi
2,6-di-cyclopentyl-4-methylphenol
2-(α-methylcyclohexyl)-4,6-dimethylphenol
2,6-di-octadecyl-4-methylphenol
2,4,6-tri-cyclohexylphenol
2,6-di-tert-butyl-4-methoxymethylphenol

### 1.2. Alkylated hydroquinones, for example,

2,6-di-tert-butyl-4-methoxyphenol
2,5-di-tert-butyl-hydroquinone
2,5-di-tert-amyl-hydroquinone
2,6-diphenyl-4-octadecyloxyphenol

### 1.3. Hydroxylated thiodiphenyl ethers, for example

2,2'-thio-bis-(6-tert-butyl-4-methylphenol)
2,2'-thio-bis-(4-octylphenol)
4,4'-thio-bis-(6-tert-butyl-3-methylphenol)
4,4'-thio-bis-(6-tert-butyl-2-methylphenol)

### 1.4. Alkylidene-bisphenols, for example,

2,2'-methylene-bis-(6-tert-butyl-4-methylphenol)
2,2'-methylene-bis-(6-tert-butyl-4-ethylphenol)
2,2'-methylene-bis-[4-methyl-6-(α-methylcyclohexyl)-phenol]
2,2'-methylene-bis-(4-methyl-6-cyclohexylphenol)
2,2'-methylene-bis-(6-nonyl-4-methylphenol)
2,2'-methylene-bis-[6-(α-methylbenzyl)-4-nonylphenol]
2,2'-methylene-bis-[6-(α,α-dimethylbenzyl)-4-nonylphenol)
2,2'-methylene-bis-(4,6-di-tert-butylphenol)
2,2'-ethylidene-bis-(4,6-di-tert-butylphenol)
2,2'-ethylidene-bis-(6-tert-butyl-4-isobutylphenol)
4,4'-methylene-bis-(2,6-di-tert-butylphenol)
4,4'-methylene-bis-(6-tert-butyl-2-methylphenol)
1,1-bis-(5-tert-butyl-4-hydroxy-2-methylphenyl-butane
2,6-di-(3-tert-butyl-5-methyl-2-hydroxybenzyl)-4-methyl-phenol
1,1,3-tris-(5-tert-butyl-4-hydroxy-2-methylphenyl)-butane
1,1-bis-(5-tert-butyl-4-hydroxy-2-methylphenyl)-3-n-dodecylmercaptobutane
ethyleneglycol bis-[3,3-bis-(3'-tert-butyl-4'-hydroxyphenyl)-butyrate]
di-(3-tert-butyl-4-hydroxy-5-methylphenyl)-dicyclopentadiene
di-[2-(3'-tert-butyl-2'-hydroxy-5'-methyl-benzyl)-6-tert-butyl-4-methylphenyl] terephthalate.

### 1.5. Benzyl compounds, for example,

1,3,5-tri-(3,5-di-tert-butyl-4-hydroxybenzyl)-2,4,6-trimethylbenzene
di-(3,5-di-tert-butyl-4-hydroxybenzyl) sulfide
3,5-di-tert-butyl-4-hydroxybenzyl-mercapto-acetic acid isooctyl ester
bis-(4-tert-butyl-3-hydroxy-2,6-dimethylbenzyl)dithiol terephthalate
1,3,5-tris-(3,5-di-tert-butyl-4-hydroxybenzyl) isocyanurate
1,3,5-tris-(4-tert-butyl-3-hydroxy-2,6-dimethylbenzyl) isocyanurate
3,5-di-tert-butyl-4-hydroxybenzyl-phosphoric acid dioctadecyl ester
3,5-di-tert-butyl-4-hydroxybenzyl-phosphoric acid monoethyl ester, calcium-salt

### 1.6. Acylaminophenols, for example,

4-hydroxy-lauric acid anilide
4-hydroxy-stearic acid anilide
2,4-bis-octylmercapto-6-(3,5-tert-butyl-4-hydroxyanilino)-s-triazine
octyl-N- (3,5-di-tert-butyl-4-hydroxyphenyl)-carbamate

### 1.7. Esters of β-(3,5-di-tert-butyl-4-hydroxyphenyl)propionic acid with monohydric or polyhydric alcohols, for example,

| | |
|---|---|
| methanol | diethylene glycol |
| octadecanol | triethylene glycol |
| 1,6-hexanediol | pentaerythritol |
| neopentyl glycol | tris-hydroxyethyl isocyanurate |
| thiodiethylene glycol | di-hydroxyethyl oxalic acid diamide |

### 1.8. Esters of β-(5-tert-butyl-4-hydroxy-3-methylphenyl)propionic acid with monohydric or polyhydric alcohols, for example,

| | |
|---|---|
| methanol | diethylene glycol |
| octadecanol | triethylene glycol |
| 1,6-hexanediol | pentaerythritol |
| neopentyl glycol | tris-hydroxyethyl isocyanurate |
| thiodiethylene glycol | di-hydroxyethyl oxalic acid diamide |

### 1.9. Amides of β-(3,5-di-tert-butyl-4-hydroxyphenyl)propionic acid for example,

N,N'-di-(3,5-di-tert-butyl-4-hydroxyphenylpropionyl)hexamethylenediamine
N,N'-di-(3,5-di-tert-butyl-4-hydroxyphenylpropionyl)trimethylenediamine
N,N'-di-(3,5-di-tert-butyl-4-hydroxyphenylpropionyl)hydrazine

### 1.10 Diarylamines, for example,

diphenylamine, N-phenyl-1-naphthylmnine, N-(4-tert-octylphenyl)-1-naphthylamine, 4,4'-di-tert-octyl-diphenylamine, reaction product of N-phenylbenzylamine and 2,4,4-trimethylpentene, reaction product of diphenylamine and 2,4,4-trimethylpentene, reaction product of N-phenyl-1-naphthylamine and 2,4,4-trimethylpentene.

### 2. UV absorbers and light stabilizers

2.1. 2-(2'-Hydroxyohenyl)-benzotriazoles, for example, the 5'-methyl-, 3',5'-di-tert-butyl-, 5'-tert-butyl-, 5'-(1,1,3,3-tetramethylbutyl)-, 5-chloro-3',5'-di-tert-butyl-, 5-chloro-3'-tert-butyl-5'-methyl-, 3'-sec-butyl-5'-tert-butyl-, 4'-octoxy, 3',5'-di-tert-amyl-, 3',5'-bis-(α,α-dimethylbenzyl), 3'-tert-butyl-5'-(2-(omega-hydroxyocta-(ethyleneoxy)carbonyl-ethyl)-, 3'-dodecyl-5'-methyl-, and 3'-tert-butyl-5'-(2-octyloxycarbonyl)ethyl-, and dodecylated-5'-methyl derivatives.

2.2. 2-Hydroxy-benzophenones, for example, the 4-hydroxy-, 4-methoxy-, 4-octoxy, 4-decyloxy-, 4-dodecyloxy-, 4-benzyloxy, 4,2',4'-trihydroxy- and 2'-hydroxy-4,4'-dimethoxy derivatives.

2.3. Esters of optionally substituted benzoic acids for example, phenyl salicylate, 4-tert-butylphenyl salicylate, octylphenyl salicylate, dibenzoylresorcinol, bis-(4-tert-butylbenzoyl)-resorcinol, benzoylresorcinol, 3,5-di-tert-butyl-4-hydroxybenzoic acid 2,4-di-tert-butylphenyl ester and 3,5-di-tert-butyl-4-hydroxybenzoic acid hexadecyl ester.

2.4. Acrylates, for example, α-cyano-β,β-diphenylacrylic acid ethyl ester or isooctyl ester, a-carbomethoxy-cinnamic acid methyl ester, α-cyano-β-methyl-p-methoxy-cinnamic acid methyl ester or butyl ester, α-carbomethoxy-p-methoxycinnamic acid methyl ester, N-(β-carbomethoxy-β-cyanovinyl)-2-methyl-indoline.

2.5 Nickel compounds, for example, nickel complexes of 2,2'-thio-bis-[4-(1,l,3,3-tetramethylbutyl)-phenol], such as the 1:1 or 1:2 complex, optionally with additional ligands such as n-butylamine, triethanolamine or N-cyclohexyl-diethanolamine, nickel dibutyldithiocarbamate, nickel salts of 4-hydroxy-3,5-di-tert-butylbenzylphosphonic acid monoalkyl esters, such as of the methyl, ethyl or butyl ester, nickel complexes of ketoximes such as of 2-hydroxy-4-methyl-phenyl undecyl ketoxime, nickel complexes of l-phenyl-4-lauroyl-5-hydroxy-pyrazole, optionally with additional ligands.

2.6. Sterically hindered amines, for example bis-(2,2,6,6-tetramethylpiperidyl) sebacate, bis-(1,2,2,6,6 bis-(1,2,2,6,6-pentamethylpiperidyl) sebacate, n-butyl-3,5-di-tert.butyl-4-hydroxybenzyl malonic acid bis-(1,2,2,6,6-pentamethylpiperidyl)ester, condensation product of 1-hydroxyethyl-2,2,6,6-tetramethyl-4-hydroxypiperidine and succinic acid, condensation product of N,N'-(2,2,6,6-tetramethylpiperidyl)-hexamethylenediamine and 4-tert-octylamino-2,6-dichloro-s-triazine, tris-(2,2,6,6-tetramethylpiperidyl)-nitrilotriacetate, tetrakis-(2,2,6,6-tetramethyl-4-piperidyl)-1,2,3,4-butanetetracarbonic acid, 1,1'(1,2-ethanediyl)-bis-(3,3,5,5-tetramethylpiperazinone, bis(1-octyloxy-2,2,6,6-tetramethylpiperidin-4-yl) sebacate.

2.7. Oxalic acid diamides, for example, 4,4'-di-octyloxyoxanilide, 2,2'-di-octyloxy-5,5'-di-tert-butyl-oxanilide, 2,2'-di-dodecyloxy-5,5'-di-tert-butyl-oxanilide, 2-ethoxy-2'-ethyl-oxanilide, N,N'-bis (3-dimethylaminopropyl)oxalamide, 2-ethoxy-5-tert-butyl-2'-ethyloxanilide and its mixture with 2-ethoxy-2'-ethyl-5,4'-di-tert-butyloxanilide and mixtures of ortho- and para-methoxy-as well as of o- and p-ethoxy-disubstituted oxanilides.

2.8. Hydroxyphenyl-s-triazines, for example 2,6-bis-(2,4-dimethylphenyl)-4-(2-hydroxy-4-octyloxyphenyl)-s-triazine; 2,6-bis-(2,4-dimethylphenyl)-4-(2,4-dihydroxyphenyl)-s-triazine; 2,4-bis(2,4-dihydroxyphenyl)-6-(4-chlorophenyl)-s-triazine; 2,4-bis[2-hydroxy-4-(2-hydroxyethoxy)phenyl]-6-(4-chlorophenyl)-s-triazine; 2,4-bis[2-hydroxy-4-(2-hydroxyethoxy)phenyl]-6-phenyl-s-triazine; 2,4-bis[2-hydroxy-4-(2-hydroxyethoxy)phenyl]-6-(2,4-dimethylphenyl)-s-triazine; 2,4-bis[2-hydroxy-4-(2-hydroxyethoxy)phenyl]-6-(4-bromophenyl)-s-triazine; 2,4-bis[2-hydroxy-4-(2-acetoxyethoxy)phenyl]-6-(4-chlorophenyl)-s-triazine, 2,4-bis(2,4-dihydroxyphenyl)-6-(2,4-dimethylphenyl)-s-triazine.

3. Metal deactivators, for example, N,N'-diphenyloxalic acid diamide, N-salicylal-N'-salicyloylhydrazine, N,N'-bis-salicyloylhydrazine, N,N'-bis-(3,5-di-tert-butyl-4-hydroxyphenylpropionyl)-hydrazine, 3-salicyloylamino-1,2,4-triazole, bis-benzylidene-oxalic acid dihydrazide.

4. Phosphites and phosphonites, for example, triphenyl phosphite, diphenylalkyl phosphites, phenyldialkyl phosphites, tri-(nonylphenyl) phosphite, trilauryl phosphite, trioctadecyl phosphite, di-stearyl-pentaerythritol diphosphite, tris-(2,4-di-tert-butylphenyl) phosphite, di-isodecylpentaerythritol diphosphite, di-(2,4-di-tert-butylphenyl)pentaerythritol diphosphite, tristearylsorbitol triphosphite, tetrakis-(2,4-di-tert-butylphenyl) 4,4'-diphenylylenediphosphonite.

5. Compounds which destroy peroxide, for example, esters of β-thiodipropionic acid, for example the lauryl, stearyl, myristyl or tridecyl esters, mercapto-benzimidazole or the zinc salt of 2-mercaptobenzimidazole, zinc dibutyl-dithiocarbamate, dioctadecyl disulfide, pentaerythritol tetrakis-(β-dodecylmercapto)-propionate.

6. Hydroxylamines, for example, N,N-dibenzylhydroxylamine, N,N-diethylhydroxylamine, N,N-dioctylhydroxylamine, N,N-dilaurylhydroxylamine, N,N-ditetradecylhydroxylamine, N,N-dihexadecylhydroxylamine, N,N-dioctadecylhydroxylamine, N-hexadecyl-N-octadecylhydroxylamine, N-heptadecyl-N-octadecylhydroxylamine, N,N-dialkylhydroxylamine derived from hydrogenated tallow amine.

7. Nitrones, for example, N-benzyl-alpha-phenyl nitrone, N-ethyl-alpha-methyl nitrone, N-octyl-alpha-heptyl nitrone, N-lauryl-alpha-undecyl nitrone, N-tetradecyl-alpha-tridecyl nitrone, N-hexadecyl-alpha-pentadecyl nitrone, N-octadecyl-alpha-heptadecylnitrone, N-hexadecyl-alpha-heptadecyl nitrone, N-octadecyl-alpha-pentadecyl nitrone, N-heptadecyl-alpha-heptadecyl nitrone, N-octadecyl-alpha-hexadecyl nitrone, nitrone derived from N,N-dialkylhydroxylamine derived from hydrogenated tallow amine.

8. Polyamide stabilizers, for example copper salts in combination with iodides and/or phosphorus compounds and salts of divalent manganese.

9. Basic co-stabilizers, for example, melamine, polyvinylpyrrolidone, dicyandiamide, triallyl cyanurate, urea derivatives, hydrazine derivatives, amines, polyamides, polyurethanes, alkali metal salts and alkaline earth metal salts of higher fatty acids for example Ca stearate, Zn stearate, Mg stearate, Na ricinoleate and K palmitate, antimony pyrocatecholate or zinc pyrocatecholate.

10. Nucleating agents, for example, 4-tert-butyl-benzoic acid, adipic acid, diphenylacetic acid.

11. Fillers and reinforcing agents, for example, calcium carbonate, silicates, glass fibers, asbestos, talc, kaolin, mica, barium sulfate, metal oxides and hydroxides, carbon black, graphite.

12. Other additives, for example, plasticizers, lubricants, emulsifiers, pigments, optical brighteners, flameproofing agents, anti-static agents, blowing agents and thiosynergists such as dilauryl thiodipropionate or distearyl thiodipropionate.

### Example 1: O-Allyl-N,N-dioctadecylhydroxylamine

A mixture of 42.4 g (80 mmol) of N,N-dioctadecylhydroxylamine, 13 ml (150 mmol) of allyl bromide and 17.0 g (160 mmol) of sodium carbonate in 450 ml of ethanol is heated under reflux at approximately 80 to 85°C. The reaction is considered complete after 14 hours upon complete disappearance of the N,N-dioctadecylhydroxylamine as indicated by TLC analysis (silica gel, chloroform solvent). The reaction mixture is concentrated under reduced pressure and the resultant residue is triturated with 500 ml of chloroform. The insoluble salts are removed by filtration and the filtrate concentrated. The resultant residue is purified by flash chromatography (silica gel, chloroform solvent) to give 16.6 g (36 % yield) of the title compound as a white solid melting at 60-62°C.

| Analysis: | | | |
|---|---|---|---|
| Calcd for C₃₉H₇₉NO | C, 81.0; | H, 13.8; | N, 2.4. |
| Found | C, 80.8; | H, 13.8; | N, 2.3. |

### Example 2: O-Allyl-N,N-dioctadecylhydroxylamine

Into a cooled solution of 5.0 g (9 mmol) of N-allyl-N,N-dioctadecylamine in 50 ml of methylene chloride at 0°C under a nitrogen atmosphere is added dropwise with stirring a solution of 1.54 g (9 mmol) of 85 % active m-chloroperbenzoic acid in 50 ml of methylene chloride. After the addition is complete (approximately 15 minutes), the resultant solution is allowed to warm to ambient temperature (about 22-24°C). The reaction is complete after about 5 hours when TLC analysis (silica gel, 19:1 v/v ethyl acetate:methanol) indicates the starting material has completely reacted. The reaction mixture is transferred to a separatory funnel and washed sequentially with three 50 ml portions of 10 % aqueous sodium hydroxide solution and then with 100 ml of distilled water. The organic layer is dried over anhydrous sodium sulfate and heated under reflux at approximately 40°C for two hours. The resultant solution is concentrated in vacuo and the residue is purified by flash chromatography (silica gel, 94:6 v/v hexane:ethyl acetate eluent) to give 3.3 g (64 % yield) of the title compound as a white solid melting at 62-65°C.

| Analysis: | | | |
|---|---|---|---|
| Calcd for C₃₉H₇₉NO | C, 81.0; | H, 13.8; | N, 2.4. |
| Found | C, 80.7; | H, 14.0; | N, 2.3. |

### Example 3: O-Allyl-N,N-bis(hydrogenated tallow)hydroxylamine

Following the general procedure of Example 1 and using the hydroxylamine prepared from hydrogenated tallow amine, the above-named compound is prepared by reacting 42.4 g (80 mmol) of N,N-bis(hydrogenated tallow)hydroxylamine, 6.93 ml (80 mmol) of allyl bromide, 17.0 g (160 mmol) of sodium carbonate and 450 ml of ethanol.

The reaction residue is purified by flash chromatography (silica gel, 25:4 v/v hexane:chloroform eluent) to give 29.3 g (67 % yield) of the title compound as a white solid melting at 50-53°C.

| Analysis: | | | |
|---|---|---|---|
| Calcd for C₃₇H₇₅NO | C, 80.8; | H, 13.7; | N, 2.6. |
| Found | C, 80.9; | H, 13.8; | N, 2.5. |

### Example 4: O-Allyl-N,N-dibenzylhydroxylamine

Following the procedure of Example 1, 41.0 g (190 mmol) of N,N-dibenzylhydroxylamine, 16.5 ml (190 mmol) of allyl bromide, 30.0 g (220 mmol) of potassium carbonate and 450 ml of ethanol are reacted.

The reaction residue is purified by flash chromatography (silica gel, 49:1 v/v hexane:ethyl acetate eluent) to give 27.1 g (56 % yield) of the title compound as a colorless liquid.

| Analysis: | | | |
|---|---|---|---|
| Calcd for C₁₇H₁₉NO | C, 80.6; | H, 7.6; | N, 5.5. |
| Found | C, 80.5; | H, 7.7; | N, 5.9. |

### Example 5: O-Allyl-N,N-didecylhydroxylamine

Following the general procedure of Example 2, the above-named compound is prepared by reacting 18.0 g (53 mmol) of N-allyl-N,N-didecylamine, 9.2 g (53 mmol) of 85 % active m-chloroperbenzoic acid and 350 ml of methylene chloride.

The reaction residue is purified by flash chromatography (silica gel, 97:3 v/v hexane:ethyl acetate eluent) to give 12.8 % (68 % yield) of the title compound as a colorless oil.

| Analysis: | | | |
|---|---|---|---|
| Calcd for C₂₃H₄₇NO | C, 78.1; | H, 13.4; | N, 4.0. |
| Found | C, 77.7; | H, 13.6; | N, 4.2. |

### Example 6: O,O'-But-2-en-1,4-diyl-bis(N,N-dibenzylhydroxylamine)

In a 1000 ml three-necked flash fitted with a thermometer, an addition funnel and a condenser topped with a nitrogen sweep is charged 6.0 g (120 mmol) of a 50 % oil dispersion of sodium hydride. The sodium hydride is washed twice with 20 ml-portions of pentane to remove the oil. The sodium hydride is then suspended in 100 ml of tetrahydrofuran. Into the resultant suspension, which is now cooled to -5°C, is added dropwise a solution of 25.0 g (120 mmol) of N,N-dibenzylhydroxylamine in 150 ml of tetrahydrofuran. After the addition is complete, the reaction mixture is heated under reflux at 50 to 60°C till gas (hydrogen) evolution ceases (this takes approximately six hours). The reaction mixture is then cooled in an ice water bath and a solution of 12.8 g (60 mmol) of 1,4-dibromo-2-butene in 60 ml of tetrahydrofuran is added to the reaction mixture maintaining the reaction temperature at about 5°C during the addition step. After the addition is complete, the reaction mixture is heated under reflux at 50-60°C for six hours. The cooled reaction mixture is then treated with 100 ml of water. The resultant layers are separated and the organic layer is dried over anhydrous magnesium sulfate. Evaporation of the solvent gives 24.5 g of a crude yellow solid residue. A 15.0 g-portion of said residue is purified by HPLC (silica gel, 95:5 v/v hexane:ethyl acetate eluent) to give 1.42 g of a viscous yellow oil which was crystallized from hexane to give 0.87 g of the title compound as a white solid melting at 82-85°C.

| Analysis: | | | |
|---|---|---|---|
| Calcd for C₃₂H₃₄N₂O₂ | C, 80.3; | H, 7.2; | N, 5.9. |
| Found | C, 80.3; | H, 7.2; | N, 5.6. |

### Example 7: O,O'-2-Methylenepropan-1,3-diyl-bis(N,N-dibenzylhydroxylamine )

Following the procedure of Example 6, the above-named compound is prepared using 25.0 g (120 mmol) of N,N-dibenzylhydroxylamine, 6.7 g (140 mmol) of a 50 % oil dispersion of sodium hydride, 7.5 g (60 mmol) of 3-chloro-2-chloromethyl-1-propene and 220 ml of tetrahydrofuran to give 27.15 g of a crude reaction mixture as a yellow solid.

A 6.0 g-portion of said yellow solid is purified by HPLC (silica gel, 97:3 v/v hexane:ethyl acetate eluent) to give 2.92 g of the title compound as a yellowish viscous liquid.

Mass spectrum (m/z 478) and ¹HNMR spectral data are consistent with the structure of the title compound.

### Example 8: O,O'-But-2-en-1,4-diyl-bis(N,N-diphenylhydroxylamine)

Following the general procedure of Example 6, the above-named compound is prepared using 25.0 g (135 mmol) of N,N-diphenylhydroxylamine, 6.7 g (140 mmol) of a 50 % oil dispersion of sodium hydride, 14.4 g (68 mmol) of 1,4-dibromo-2-butene and 200 ml of tetrahydrofuran to give a crude reaction mixture which is purified by flash chromatography (silica gel, 95:5 v/v hexane:ethyl acetate eluent) to give 11.4 g of a semi-solid. This semi-solid is further purified by recrystallization from 75 ml of hot hexane to give 4.5 g of the title compound as a solid melting at 74-76°C.

| Analysis: | | | |
|---|---|---|---|
| Calcd for C₂₈H₂₆N₂O₂: | C, 79.6; | H, 6.2; | N, 6.6. |
| Found | C, 79.6; | H, 6.1; | N, 6.5. |

### Example 9: O-Allyl-N,N-diphenylhydroxylamine

Following the general procedure of Example 6, the above-named compound is prepared from N,N-diphenylhydroxylamine and allyl bromide.

### Example 10: N-Allyl-N,N-dioctadecylamine-N-oxide

Into a cooled solution of 5.0 g (9 mmol) of N-allyl-N,N-dioctadecylamine in 50 ml of chloroform at 0°C under a nitrogen atmosphere is added dropwise with stirring a solution of 2.82 g (9 mmol) of 55 % active m-chloroperbenzoic acid in 25 ml of chloroform. After the addition is complete (approximately 10 minutes), the resultant solution is allowed to warm to ambient temperature (about 22-24°C). The reaction is complete after about 2 hours when TLC analysis (silica gel, 19:1 v/v ethyl acetate:methanol)indicates the starting material has completely reacted. The reaction mixture is passed through a short column of basic alumina using chloroform as an eluent to give 5.2 g (quantitative yield) of the title compound as a white solid melting at 59-64°C.

TLC and ¹HNMR analyses confirm the structure of the title compound.

### Example 11: N-Allyl-N,N-bis(hydrogenated tallow)amine-N-oxide

Following the general procedure of Example 10 and using an equivalent amount of N-allyl-N,N-bis(hydrogenated tallow)amine in place of N-allyl-N,N-dioctadecylamine the title compound is prepared.

### Example 12: N-Allyl-N,N-dibenzylamine-N-oxide

Following the general procedure of Example 10, the title compound is prepared starting with N-allyl-N,N-dibenzylamine as starting material.

### Example 13: N-Allyl-N,N-dioctylamine-N-oxide

The title compound is prepared following the general procedure of Example 10 starting from N-allyl-N,N-dioctylamine.

### Example 14: N-Allyl-N,N-diphenylamine-N-oxide

The title compound is prepared starting with N-allyl-N,N-diphenylamine using the general procedure of Example 10.

### Example 15: N,N'-But-2-en-1,4-diyl -bis (N,N-dibenzylamine)-N,N'-dioxide

The title compound is prepared following the general procedure of Example 10 starting with N,N'-but-2-en-1,4-diyl-bis(N,N-dibenzylamine).

### Example 16: N,N'-But-2-en-1,4-diyl-bis(N,N-diphenylamine)-N,N'-dioxide

The title compound is prepared following the general procedure of Example 10 starting with N,N'-but-2-en-1,4-diyl-bis(N,N-diphenylamine).

### Example 17: Process Stabilization of Polypropylene at 280°C

The test stabilizers are solvent blended using methylene chloride into unstabilized polypropylene (®PROFAX 6501 Himont) which already contains 0.1 % by weight of calcium stearate. After removal of solvent by evaporation under reduced pressure, the resin is extruded using an MPM one-inch single screw extruder under the following extruder conditions:

| | |
|---|---|
| Screw RPM | 80 |
| Cylinder #1 heater zone | 243°C |
| Cylinder #2 heater zone | 268°C |
| Cylinder #3 heater zone | 279°C |
| Gate, adapter die | 282°C |
| Melt Temperature | 280-283°C |
| Residence Time seconds | 45 |

After the first and fifth extrusions, the melt flow rate (MFR) is determined by the ASTM method 1238 condition L. The MFR of polypropylene varies inversely with molecular weight. The higher is the MFR, , the lower is the molecular weight. A relative change in MFR between the first and fifth extrusion values indicates that the relative effectiveness of the test stabilizer in protecting the polypropylene from degradation during processing at the elevated temperature. The MFR data for a number of test stabilizers are given in the tables below.

| Additive Compound of | Additive Concentration (% by weight) | MFR (g/10 min) after Extrusion | |
|---|---|---|---|
| | | 1 | 5 |
| None** | -- | 17.3 | 102 |
| Example 1 | 0.1 | 5.3 | 10.0 |
| Example 5 | 0.1 | 6.4 | 9.3 |
| AO A plus | 0.1 | | |
| Example 1 | 0.05 | 5.1 | 9.1 |

| | | | |
|---|---|---|---|
| AO A is neopentanetetrayl tetrakis(3,5-di-tert-butyl-4-hydroxyhydrocinnamate). ** Base resin contains 0.1% by weight of calcium stearate. | | | |

| Additive Compound of | Additive Concentration (% by weight) | MFR (g/10 min) after Extrusion | |
|---|---|---|---|
| | | 1 | 5 |
| AO A | 0.1 | 17.9 | 25.6 |
| AO A plus | 0.1 | | |
| Example 10 | 0.05 | 8.6 | 12.5 |
| AO A is neopentanetetrayl tetrakis(3,5-di-tert-butyl-4-hydroxyhydrocinnamate). | | | |
| Base resin contains 0.1 % by weight of calcium stearate. | | | |

### Example 18: Color Stabilization of Polypropylene

This example illustrates the color stabilizing effectiveness of the instant compounds in combination with a phenolic antioxidant.

When pellets obtained after the first and fifth extrusion as described in Example 17 are compression molded into 125 mil (3.2 mm) thick plaques at 193°C, the yellowness index (YI) values on said plaques are determined according to ASTM test method D 1925. The results are given below.

| Additive Compound of | Additive Concentration (% by weight) | Yellowness Index after Extrusion | |
|---|---|---|---|
| | | 1 | 5 |
| AO A plus | 0.1 | | |
| Example 1 | 0.05 | 8.4 | 15.5 |

| Additive Compound of | Additive Concentration (% by weight) | Yellowness Index after Extrusion |
|---|---|---|
| | | 5 |
| AO A plus | 0.1 | |
| Example 10 | 0.05 | 13.5 |
| AO A is neopentanetetrayl tetrakis(3,5-di-tert-butyl-4-hydroxyhydrocinnamate). | | |

### Example 19:

### Standard Test Method for Oxidation Stability of Gasoline Automotive Engine Oils by Thin-Film Oxygen Uptake (TFOUT)

The antioxidant effectiveness of the instant stabilizers in engine oils is evaluated by the ASTM test method, D4742. A 1.5 gram test sample of a 10W-30 engine oil, formulated to meet SD/CC quality level, containing 0.5**%** by weight of the test compound is placed in the test apparatus. The test is then completed according to the standard method procedure and the oxidation induction time, in minutes, is reported in the table below. A longer induction time indicates greater oxidation stability.

| Test Compound of | Oxidation Induction Time (minutes) |
|---|---|
| Base Oil (no stabilizer) | 110 |
| Example 8 | 218 |

The instant O-alkenyl substituted hydroxylamine is an effective antioxidant for engine oils.

### Example 20:

When using the test procedure of Example 19 the O-alkenyl compound of Example 8 is replaced by the instant compound of Example 9, the O-allyl-N,N-diphenylhydroxylamine is shown to be an effective antioxidant for engine oils.

### Example 21:

When using the test procedure of Example 19 the compound of Example 8 is replaced by the instant compound of Example 14 or 16, the compound of Example 14 or 16 is also shown to be an effective antioxidant for engine oils.

## Claims

1. A stabilized composition which comprises
(a) a synthetic polymer or a lubricant subject to oxidative, thermal or actinic-induced degradation, and
(b) 0.01 to 5 % by weight of the stabilized composition of a compound of formula I 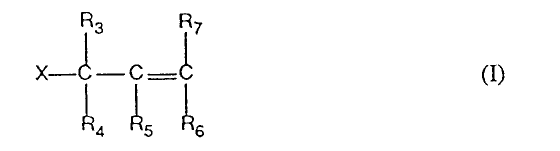
wherein X is a group of formula Ia or Ib, 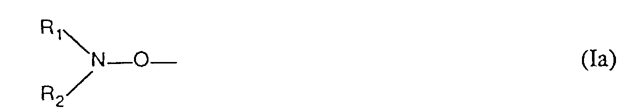 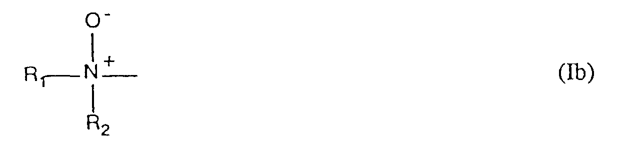
R₁ and R₂ are independently hydrogen, a straight or branched chain alkyl of 1 to 36 carbon atoms, alkyl of 1 to 18 carbon atoms terminated by a group -OR₈, -NR₉R₁₀, -SR₁₁, -COOR₁₂ or -CONR₁₃R₁₄ or interrupted by arylene of 6 to 10 carbon atoms, -O-, -S-, -SO-, -SO₂-, -COO-, -OCO-, -CONR₁₅-, -NR₁₅CO- or -NR₁₆- where R₈, R₉, R₁₀, R₁₁, R₁₂, R₁₃, R₁₄, R₁₅ and R₁₆ are independently hydrogen, alkyl of 1 to 18 carbon atoms or alkenyl of 3 to 6 carbon atoms; or R₁ and R₂ are independently cycloalkyl of 5 to 12 carbon atoms, phenylalkyl of 7 to 15 carbon atoms, said phenylalkyl substituted on the phenyl ring by alkyl of 1 to 18 carbon atoms or by α-cumyl; aryl of 6 to 14 carbon atoms or said aryl substituted by one or two alkyl of 1 to 24 carbon atoms,
R₃ and R₄ are independently hydrogen, alkyl of 1 to 9 carbon atoms, said alkyl substituted by -OH or by acetoxy; alkenyl of 3 to 6 carbon atoms or aryl of 6 to 10 carbon atoms, and
R₅, R₆ and R₇ are independently hydrogen, alkyl of 1 to 9 carbon atoms, said alkyl substituted by -OH or by acetoxy; alkenyl of 3 to 6 carbon atoms, aryl of 6 to 10 carbon atoms, or, when X is a group of formula Ia, a group of formula IIa 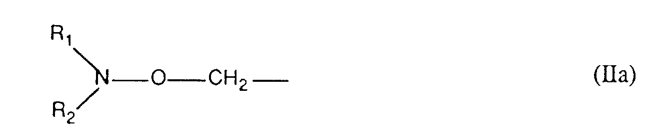 or, when X is a group of formula Ib, a group of formula IIb, 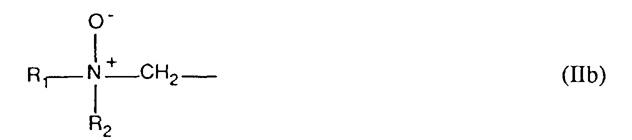 R₃ and R₄, or R₃ and R₅, or R₃ and R₇, or R₄ and R₅, or R₄ and R₇, or R₅ and R₆, or R₆ and R₇ together are a straight or branched chain alkylene of 2 to 8 carbon atoms to form a cycloalkyl or cycloalkenyl ring with 5 or 6 ring atoms.

2. A composition according to claim 1 wherein the synthetic polymer is a polyolefin.

3. A composition according to claim 2 wherein the polyolefin is polypropylene.

4. A composition according to claim 1 wherein the lubricant is a motor oil.

5. A composition according to claim 4 wherein the motor oil is a gasoline engine oil.

6. A composition according to claim 1 where in the compound of formula I
R₁ and R₂ are independently a straight or branched chain alkyl of 1 to 20 carbon atoms, alkyl of 1 to 18 carbon atoms terminated by a group -OR₈, -NR₉R₁₀, -SR₁₁, -COOR₁₂ or -CONR₁₃R₁₄ or interrupted by phenylene, -O-, -S-, -SO-, -SO₂-, -COO-, -OCO-, -CONR₁₅-, -NR₁₅CO- or -NR₁₆- wherein R₈, R₉, R₁₀, R_{1 1}, R₁₂,R ₁₃, R₁₄, R₁₅ and R₁₆ are independently hydrogen or alkyl of 1 to 18 carbon atoms; or R₁ and R₂ are cycloalkyl of 5 to 7 carbon atoms, benzyl, benzyl substituted on the phenyl ring by alkyl of 1 to 12 carbon atoms or by α-cumyl, aryl of 6 to 14 carbon atoms or said aryl substituted by one or two alkyl of 1 to 24 carbon atoms,
R₃ is hydrogen or alkyl of 1 to 4 carbon atoms,
R₄ is hydrogen, alkyl of 1 to 4 carbon atoms or phenyl,
R₅ and R₆ are independently hydrogen, alkyl of 1 to 9 carbon atoms, or, when X is a group of formula Ia, a group of formula IIa or, when X is a group of formula Ib, a group of formula IIb,
R₇ is hydrogen, alkyl of 1 to 9 carbon atoms or phenyl.

7. A composition according to claim 6 wherein R₁ and R₂ are independently alkyl of 8 to 18 carbon atoms, cyclohexyl, benzyl, phenyl, 1-naphthyl or said phenyl or said naphthyl substituted by one or two alkyl of 4 to 12 carbon atoms,
R₃ is hydrogen,
R₄ is hydrogen or methyl,
R₅ and R₆ are independently hydrogen or, when X is a group of formula Ia, a group of formula IIa or, when X is a group of formula Ib, a group of formula IIb,
R₇ is hydrogen.

8. A composition according to claim 1 wherein component (b) is O-allyl-N,N-dioctadecylhydroxylamine, O-allyl-N,N-dibenzylhydroxylamine, O,O'-but-2-en-1,4-diyl-bis(N,N-diphenylhydroxylamine), O-allyl-N,N-diphenylhydroxylamine or N-allyl-N,N-dioctadecylamine-N-oxide.

9. A compound of formula III 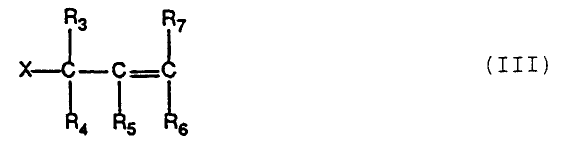 wherein X is a group of formula IIIa or IIIb, 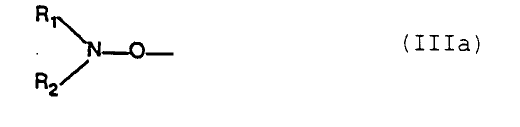 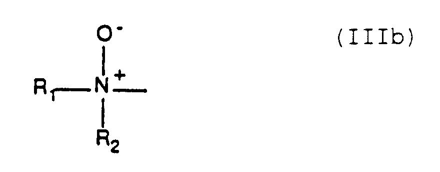
R₁ and R₂ are independently alkyl of 8 to 36 carbon atoms, benzyl, benzyl substituted on the phenyl ring by alkyl of 1 to 18 carbon atoms or by α-cumyl; aryl of 6 to 14 carbon atoms or said aryl substituted by one or two alkyl of 1 to 24 carbon atoms,
R₃ and R₄ are independently hydrogen, alkyl of 1 to 9 carbon atoms, alkenyl of 3 to 6 carbon atoms or aryl of 6 to 10 carbon atoms, and
R₅, R₆ and R₇ are independently hydrogen, alkyl of 1 to 9 carbon atoms, alkenyl of 3 to 6 carbon atoms or aryl of 6 to 10 carbon atoms or, when X is a group of formula IIIa, a group of formula IVa 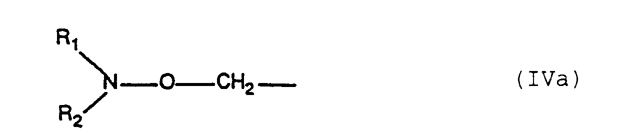 or, when X is a group of formula IIIb, a group of formula IVb,

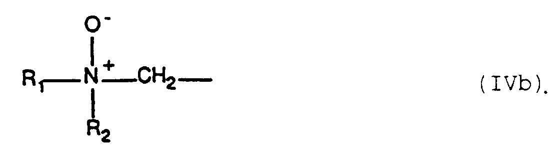

10. A compound according to claim 9 wherein
R₁ and R₂ are independently alkyl of 8 to 18 carbon atoms, benzyl, phenyl, 1-naphthyl or said phenyl or said naphthyl substituted by one or two alkyl of 4 to 12 carbon atoms,
R₃ is hydrogen,
R₄ is hydrogen or methyl,
R₅ and R₆ are independently hydrogen or, when X is a group of formula IIIa a group of formula IVa or, when X is a group of formula IIIb, a group of formula IVb, and
R₇ is hydrogen.

11. A compound according to claim 10 wherein
R₁ and R₂ are independently alkyl of 12 to 18 carbon atoms, benzyl, phenyl, 1-naphthyl, or said phenyl or said naphthyl substituted by one or two alkyl of 4 to 8 carbon atoms,
R₃ is hydrogen,
R₄ is hydrogen,
R₅ is hydrogen or, when X is a group of formula IIIa, a group of formula IVa or, when X is a group of formula IIIb, a group of formula IVb,
R₆ is hydrogen or, when X is a group of formula IIIa, a group of formula IVa or, when X is a group of formula IIIb, a group of formula IVb, and
R₇ is hydrogen.

12. The compound according to claim 9 which is
O-allyl-N,N-dioctadecylhydroxylamine,
O-allyl-N,N-di(hydrogenated tallow)hydroxylamine,
O-allyl-N,N-dibenzylhydroxylamine,
O-allyl-N,N-didecylhydroxylamine,
O,O'-but-2-en-1,4-diyl-bis(N,N-dibenzylhydroxylamine),
O,O'-2-methylenepropan-1,3-diyl-bis(N,N-dibenzylhydroxylamine),
O,O'-but-2-en-1,4-diyl-bis(N,N-diphenylhydroxylamine),
O-allyl-N,N-diphenylhydroxylamine,
N-allyl-N,N-dioctadecylamine-N-oxide,
N-allyl-N,N-di(hydrogenated tallow)amine-N-oxide,
N-allyl-N ,N-dibenzylamine-N-oxide,
N-allyl-N,N-dioctylamine-N-oxide,
N,N'-but-2-en-1,4-diyl-bis(N,N-dibenzylamine)-N,N'-dioxide,
N,N'-but-2-en-1,4-diyl-bis-(N,N-diphenylamine)-N,N'-dioxide or
N-allyl-N ,N-diphenylamine-N-oxide.

## Patentansprüche

1. Stabilisierte Zusammensetzung, umfassend
(a) ein synthetisches Polymer oder ein Gleitmittel, das einem oxidativen, thermischen oder strahlungsbedingten Abbau unterliegt, und
(b) 0,01 bis 5 Gew.% der stabilisierten Zusammensetzung einer Verbindung der Formel I 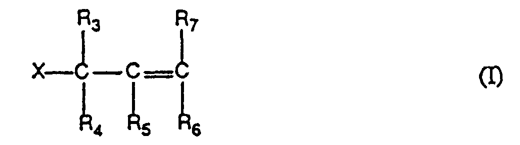
worin X für eine Gruppe der Formel Ia oder Ib 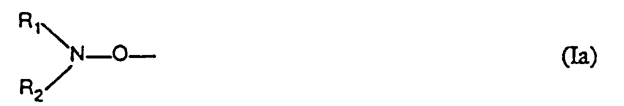 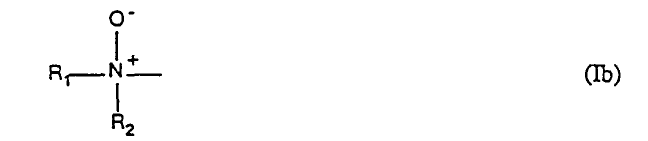 steht,
R₁ und R₂ unabhängig Wasserstoff, geradkettiges oder verzweigtes Alkyl mit 1 bis 36 Kohlenstoffatomen, Alkyl mit 1 bis 18 Kohlenstoffatomen, terminiert durch eine Gruppe -OR₈, -NR₉R₁₀, -SR₁₁, -COOR₁₂ oder -CONR₁₃R₁₄ oder Unterbrochen durch Arylen mit 6 bis 10 Kohlenstoffatomen, -O-, -S-, -SO-, -SO₂-, -COO-, -OCO-, -CONR₁₅-, -NR₁₅CO- oder -NR₁₆- bedeuten, worin R₈, R₉, R₁₀, R₁₁, R₁₂, R₁₃, R₁₄, R₁₅ und R₁₆ unabhängig Wasserstoff, Alkyl mit 1 bis 18 Kohlenstoffatomen oder Alkenyl mit 3 bis 6 Kohlenstoffatomen wiedergeben; oder R₁ und R₂ unabhängig Cycloalkyl mit 5 bis 12 Kohlenstoffatomen, Phenylalkyl mit 7 bis 15 Kohlenstoffatomen, besagtes Phenylalkyl substituiert an dem Phenylring durch Alkyl mit 1 bis 18 Kohlenstoffatomen oder durch α-Cumyl; Aryl mit 6 bis 14 Kohlenstoffatomen oder besagtes Aryl substituiert durch ein oder zwei Alkyl mit 1 bis 24 Kohlenstoffatomen bedeuten,
R₃ und R₄ unabhängig Wasserstoff, Alkyl mit 1 bis 9 Kohlenstoffatomen, besagtes Alkyl substituiert durch -OH oder Acetoxy; Alkenyl mit 3 bis 6 Kohlenstoffatomen oder Aryl mit 6 bis 10 Kohlenstoffatomen bedeuten, und R₅, R₆ und R₇ unabhängig Wasserstoff, Alkyl mit 1 bis 9 Kohlenstoffatomen, besagtes Alkyl substituiert durch -OH oder durch Acetoxy; Alkenyl mit 3 bis 6 Kohlenstoffatomen, Aryl mit 6 bis 10 Kohlenstoffatomen oder, wenn X eine Gruppe der Formel Ia ist,eine Gruppe der Formel IIa 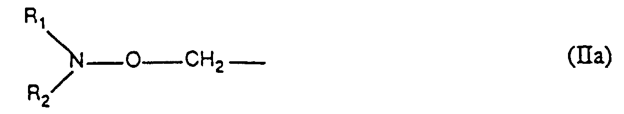 oder, wenn X für eine Gruppe der Formel Ib steht, eine Gruppe der Formel IIb 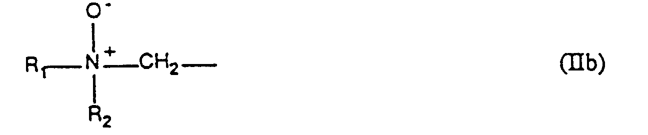 bedeuten,
R₃ und R₄ oder R₃ und R₅ oder R₃ und R₇ oder R₄ und R₅ oder R₄ und R₇ oder R₅ und R₆ oder R₆ und R₇ gemeinsam ein geradkettiges oder verzweigtes Alkylen mit 2 bis 8 Kohlenstoffatomen unter Bildung eines Cycloalkyl- oder Cycloalkenylrings mit 5 oder 6 Ringatomen darstellen.

2. Zusammensetzung gemäß Anspruch 1, worin das synthetische Polymere ein Polyolefin ist.

3. Zusammensetzung gemäß Anspruch 2, worin das Polyolefin Polypropylen ist.

4. Zusammensetzung gemäß Anspruch 1, worin das Gleitmittel ein Motorenöl ist.

5. Zusammensetzung gemäß Anspruch 4, worin das Motorenöl ein Benzinmotorenöl ist.

6. Zusammensetzung gemäß Anspruch 1, worin in der Verbindung der Formel I
R₁ und R₂ unabhängig geradkettiges oder verzweigtes Alkyl mit 1 bis 20 Kohlenstoffatomen, Alkyl mit 1 bis 18 Kohlenstoffatomen, terminiert durch eine Gruppe -OR₈, -NR₉R₁₀, -SR₁₁, -COOR₁₂ oder -CONR₁₃R₁₄ oder unterbrochen durch Phenylen, -O-, -S-, -SO-, -SO₂-, -COO-, -OCO-, -CONR₁₅-, -NR₁₅CO- oder -NR₁₆-, sind, worin R₈, R₉, R₁₀, R₁₁, R₁₂, R₁₃, R₁₄, R₁₅ und R₁₆ unabhängig Wasserstoff oder Alkyl mit 1 bis 18 Kohlenstoffatomen bedeuten; oder R₁ und R₂ Cycloalkyl mit 5 bis 7 Kohlenstoffatomen, Benzyl, Benzyl, substituiert an dem Phenylring durch Alkyl mit 1 bis 12 Kohlenstoffatomen oder durch α-Cumyl, Aryl mit 6 bis 14 Kohlenstoffatomen oder besagtes Aryl substituiert durch ein oder zwei Alkyl mit 1 bis 24 Kohlenstoffatomen bedeuten,
R₃ Wasserstoff oder Alkyl mit 1 bis 4 Kohlenstoffatomen ist, R₄ Wasserstoff, Alkyl mit 1 bis 4 Kohlenstoffatomen oder Phenyl bedeutet,
R₅ und R₆ unabhängig Wasserstoff, Alkyl mit 1 bis 9 Kohlenstoffatomen oder, wenn X eine Gruppe der Formel Ia ist, eine Gruppe der Formel IIa oder, wenn X eine Gruppe der Formel Ib ist, eine Gruppe der Formel IIb bedeuten,
R₇ Wasserstoff, Alkyl mit 1 bis 9 Kohlenstoffatomen oder Phenyl wiedergibt.

7. Zusammensetzung gemäß Anspruch 6, worin R₁ und R₂ unabhängig Alkyl mit 8 bis 18 Kohlenstoffatomen, Cyclohexyl, Benzyl, Phenyl, 1-Naphthyl oder besagtes Phenyl oder besagtes Naphthyl substituiert durch ein oder zwei Alkyl mit 4 bis 12 Kohlenstoffatomen sind,
R₃ Wasserstoff bedeutet,
R₄ für Wasserstoff oder Methyl steht,
R₅ und R₆ unabhängig Wasserstoff oder, wenn X eine Gruppe der Formel Ia ist, eine Gruppe der Formel IIa oder, wenn X eine Gruppe der Formel Ib ist, eine Gruppe der Formel IIb wiedergeben,
R₇ für Wasserstoff steht.

8. Zusammensetzung gemäß Anspruch 1, worin Komponente (b) O-Allyl-N,N-dioctadecylhydroxylamin, O-Allyl-N,N-dibenzylhydroxylamin, O,O'-But-2-en-1,4-diyl-bis-(N,N-diphenylhydroxylamin), O-Allyl-N,N-diphenylhydroxylamin oder N-Allyl-N,N-dioctadecylamin-N-oxid ist.

9. Verbindung der Formel III 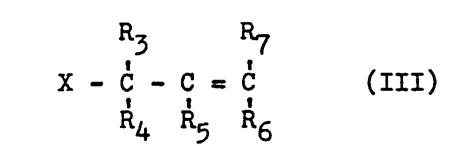 worin X eine Gruppe der Formel IIIa oder IIIb 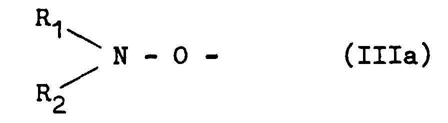 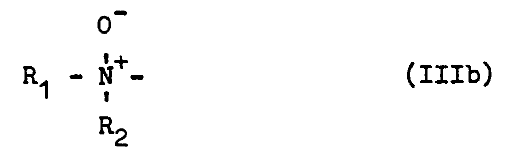 ist,
R₁ und R₂ unabhängig Alkyl mit 8 bis 36 Kohlenstoffatomen, Benzyl, Benzyl, substituiert an dem Phenylring durch Alkyl mit 1 bis 18 Kohlenstoffatomen oder durch α-Cumyl; Aryl mit 6 bis 14 Kohlenstoffatomen oder besagtes Aryl substituiert durch ein oder zwei Alkyl mit 1 bis 24 Kohlenstoffatomen wiedergeben,
R₃ und R₄ unabhängig für Wasserstoff, Alkyl mit 1 bis 9 Kohlenstoffatomen, Alkenyl mit 3 bis 6 Kohlenstoffatomen oder Aryl mit 6 bis 10 Kohlenstoffatomen stehen und
R₅, R₆ und R₇ unabhängig Wasserstoff, Alkyl mit 1 bis 9 Kohlenstoffatomen, Alkenyl mit 3 bis 6 Kohlenstoffatomen oder Aryl mit 6 bis 10 Kohlenstoffatomen oder, wenn X für eine Gruppe der Formel IIIa steht, eine Gruppe der Formel IVa 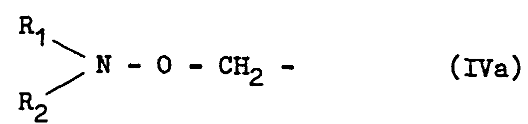 oder, wenn X für eine Gruppe der Formel IIIb steht, eine Gruppe der Formel IVb 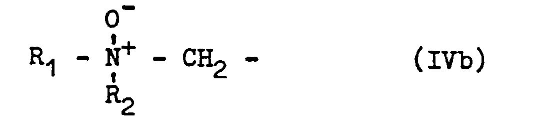 bedeuten.

10. Verbindung gemäß Anspruch 9, worin
R₁ und R₂ unabhängig Alkyl mit 8 bis 18 Kohlenstoffatomen, Benzyl, Phenyl, 1-Naphthyl oder besagtes Phenyl oder besagtes Naphthyl substituiert durch ein oder zwei Alkyl mit 4 bis 12 Kohlenstoffatomen bedeuten,
R₃ Wasserstoff ist,
R₄ für Wasserstoff oder Methyl steht,
R₅ und R₆ unabhängig Wasserstoff oder, wenn X für eine Gruppe der Formel IIIa steht, eine Gruppe der Formel IVa oder, wenn X eine Gruppe der Formel IIIb ist, eine Gruppe der Formel IVb bedeuten und
R₇ Wasserstoff ist.

11. Verbindung gemäß Anspruch 10, worin
R₁ und R₂ unabhängig Alkyl mit 12 bis 18 Kohlenstoffatomen, Benzyl, Phenyl, 1-Naphthyl oder besagtes Phenyl oder besagtes Naphthyl substituiert durch ein oder zwei Alkyl mit 4 bis 8 Kohlenstoffatomen bedeuten,
R₃ Wasserstoff ist,
R₄ für Wasserstoff steht,
R₅ Wasserstoff oder, wenn X eine Gruppe der Formel IIIa ist, eine Gruppe der Formel IVa oder, wenn X eine Gruppe der Formel IIIb ist, eine Gruppe der Formel IVb wiedergibt,
R₆ Wasserstoff oder, wenn X eine Gruppe der Formel IIIa ist, eine Gruppe der Formel IVa oder, wenn X eine Gruppe der Formel IIIb ist, eine Gruppe der Formel IVb bedeutet und
R₇ Wasserstoff ist.

12. Als Verbindung gemäß Anspruch 9:
O-Allyl-N,N-dioctadecylhydroxylamin,
O-Allyl-N,N-di-(hydrierter talg)-hydroxylamin,
O-Allyl-N,N-dibenzylhydroxylamin,
O-Allyl-N,N-didecylhydroxylamin,
O,O'-But-2-en-1,4-diyl-bis-(N,N-dibenzylhydroxylamin),
O,O'-2-Methylenpropan-1,5-diyl-bis-(N,N-dibenzylhydroxylamin),
O,O'-But-2-en-1,4-diyl-bis-(N,N-diphenylhydroxylamin), O-Allyl-N,N-diphenylhydroxylamin,
N-Allyl-N,N-dioctadecylamin-N-oxid,
N-Allyl-N,N-di-(hydrierter talg)-amin-N-oxid,
N-Allyl-N,N-dibenzylamin-N-oxid,
N-Allyl-N,N-dioctylamin-N-oxid,
N,N'-But-2-en-1,4-diyl-bis-(N,N-dibenzylamin)-N,N'-dioxid,
N,N'-But-2-en-1,4-diyl-bis-(N,N-diphenylamin),N,N'-dioxid oder
N-Allyl-N,N-diphenylamin-N-oxid.

## Revendications

1. Composition stabilisée qui comprend
(a) un polymère synthétique ou un lubrifiant sujet à une dégradation par oxydation, induite par la chaleur ou par un rayonnement actinique, et
(c) 0,01 à 5% en poids de la composition stabilisée de 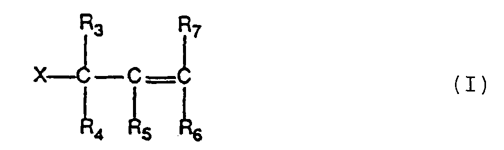
dans laquelle X est un groupe de formule Ia ou Ib 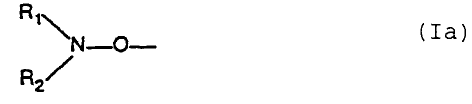 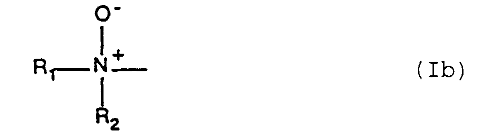
R₁ et R₂ représentent indépendamment un atome d'hydrogène, un groupe alkyle à chaîne droite ou ramifiée de 1 à 36 atomes de carbone, alkyle de 1 à 18 atomes de carbone terminé par un groupe -OR₈, -NR₉R₁₀, -SR₁₁, -COOR₁₂ ou -CONR₁₃R₁₄ ou interrompu par un groupe arylène de 6 à 10 atomes de carbone, -O-, -SO-, -SO₂-, -COO-, -OCO-, -CONR₁₅-, -NR₁₅-CO- ou -NR₁₆- où R₈, R₉, R₁₀, R₁₁, R₁₂, R₁₃, R₁₄, R₁₅ et R₁₆ représentent indépendamment un atome d'hydrogène, un groupe alkyle de 1 à 18 atomes de carbone ou alcényle de 3 à 6 atomes de carbone; ou bien R₁ et R₂ sont indépendamment un groupe cycloalkyle de 5 à 12 atomes de carbone, phénylalkyle de 7 à 15 atomes de carbone, ledit groupe phénylalkyle étant substitué sur le cycle phényle par un groupe alkyle de 1 à 18 atomes de carbone ou par un groupe α-cumyle; un groupe aryle de 6 à 14 atomes de carbone, ledit groupe aryle étant substitué par un ou deux groupes alkyle de 1 à 24 atomes de carbone,
R₃ et R₄ représentent indépendamment un atome d'hydrogène, un groupe alkyle de 1 à 9 atomes de carbone, ledit groupe alkyle étant substitué par -OH ou par un groupe acétoxy; alcényle de 3 à 6 atomes de carbone ou aryle de 6 à 10 atomes de carbone, et R₅, R₆ et R₇ représentent indépendamment un atome d'hydrogène, un groupe alkyle de 1 à 9 atomes de carbone, ledit groupe alkyle étant substitué par -OH ou par un groupe acétoxy; alcényle de 3 à 6 atomes de carbone, aryle de 6 à 10 atomes de carbone, ou, lorsque X est un groupe de formule Ia, un groupe de formule IIa 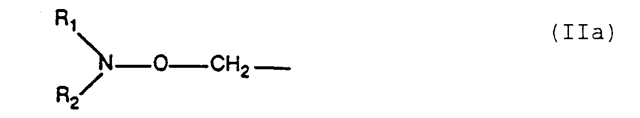 ou, lorsque X est un groupe de formule Ib, un groupe de formule IIb, 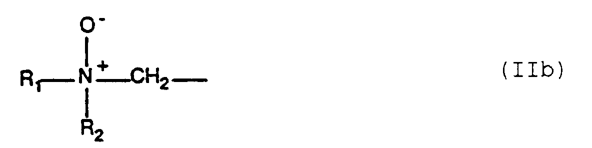
R₃ et R₄, ou R₃ et R₅, ou R₃ et R₇, ou R₄ et R₅, ou R₄ et R₇, ou R₅ et R₆, ou R₆ et R₇ représentent conjointement un groupe alkylène à chaîne droite ou ramifiée de 2 à 8 atomes de carbone en vue de former un cycle cycloalkyle ou cycloalcényle ayant 5 ou 6 atomes de cycle.

2. Composition selon la revendication 1, dans laquelle le polymère synthétique est une polyoléfine.

3. Composition selon la revendication 2, dans laquelle la polyoléfine est le polypropylène.

4. Composition selon la revendication 1, dans laquelle le lubrifiant est une huile moteur.

5. Composition selon la revendication 4, dans laquelle l'huile moteur est une huile pour moteur à essence.

6. Composition selon la revendication 1, où, dans le composé de formule I
R₁ et R₂ représentent indépendamment un groupe alkyle à chaîne droite ou ramifiée de 1 à 20 atomes de carbone, alkyle de 1 à 18 atomes de carbone terminé par un groupe -OR₈, -NR₉R₁₀, -SR₁₁, -COOR₁₂ ou -CONR₁₃R₁₄ ou interrompu par un groupe phénylène, -O-, -S-, -SO-, -SO₂-, -COO-, -OCO-, -CONR₁₅-, -NR₁₅CO- ou -NR₁₆- dans laquelle R₈, R₉, R₁₀, R₁₁, R₁₂, R₁₃, R₁₄, R₁₅ et R₁₆ représentent indépendamment un atome d'hydrogène ou un groupe alkyle de 1 à 18 atomes de carbone; ou bien R₁ et R₂ représentent un groupe cycloalkyle de 5 à 7 atomes de carbone, benzyle, benzyle substitué sur le noyau phényle par un groupe alkyle de 1 à 12 atomes de carbone ou par un groupe α-cumyle; aryle de 6 à 14 atomes de carbone, ou ledit groupe aryle étant substitué par un ou deux groupes alkyle de 1 à 24 atomes de carbone,
R₃ est un atome d'hydrogène ou un groupe alkyle de 1 à 4 atomes de carbone,,
R₄ est un atome d'hydrogène, un groupe alkyle de 1 à 4 atomes de carbone ou phényle,
R₅ et R₆ représentent indépendamment un atome d'hydrogène, un groupe alkyle de 1 à 9 atomes de carbone, ou, lorsque X est un groupe de formule Ia, un groupe de formule IIa, ou, lorsque X est un groupe de formule Ib, un groupe de formule IIb, et
R₇ est représente atome d'hydrogène, un groupe alkyle de 1 à 9 atomes de carbone ou phényle.

7. Composition selon la revendication 6, dans laquelle R₁ et R₂ représentent indépendamment un groupe alkyle de 8 à 18 atomes de carbone, cyclohexyle, benzyle, phényle, 1-naphtyle, ou ledit groupe phényle ou ledit groupe naphtyle étant substitués par un ou deux groupes alkyle de 4 à 12 atomes de carbone,
R₃ représente un atome d'hydrogène,
R₄ représente un atome d'hydrogène ou un groupe méthyle,
R₅ et R₆ représentent indépendamment un atome d'hydrogène ou, lorsque X est un groupe de formule Ia, un groupe de formule IIa, ou, lorsque X est un groupe de formule Ib, un groupe de formule IIb, et
R₇ est un atome d'hydrogène.

8. Composition selon la revendication 1, dans laquelle le composant (b) est la O-allyl-N,N-dioctadécylhyroxylamine, la O-allyl-N,N-dibenzylhydroxylamine, la O,O'-but-2-èn-1,4-diyl-bis(N,N-diphénylhydroxylamine), la O-allyl-N,N-diphénylhydroxylamine ou la N-allyl-N,N-dioctadécylamine-N-oxyde.

9. Composé de formule III 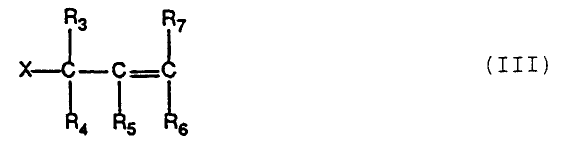 dans laquelle X est un groupe de formule IIIa ou IIIb, 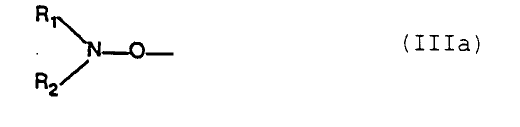 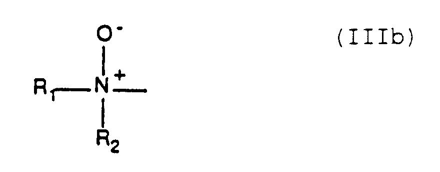
R₁ et R₂ représentent indépendamment un groupe alkyle de 8 à 36 atomes de carbone, benzyle, benzyle substitué sur le noyau phényle par un groupe alkyle de 1 à 18 atomes de carbone ou par un groupe α-cumyle; aryle de 6 à 14 atomes de carbone ou ledit groupe aryle étant substitué par un ou deux groupes alkyle de 1 à 24 atomes de carbone,
R₃ et R₄ représentent indépendamment un atome d'hydrogène, un groupe alkyle de 1 à 9 atomes de carbone, alcényle de 3 à 6 atomes de carbone ou aryle de 6 à 10 atomes de carbone, et
R₅, R₆ et R₇ représentent indépendamment un atome d'hydrogène, un groupe alkyle de 1 à 9 atomes de carbone, alcényle de 3 à 6 atomes de carbone, ou aryle de 6 à 10 atomes de carbone ou, lorsque X est un groupe de formule IIIa, un groupe de formule IVa 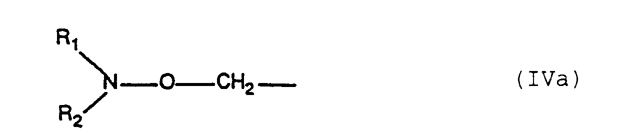 ou, lorsque X est un groupe de formule IIIb, un groupe de formule IVb

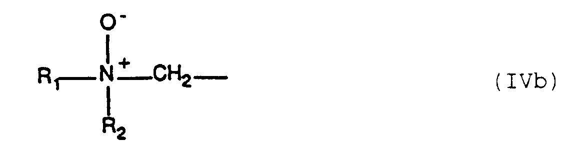

10. Composés selon la revendication 9, dans lequel
R₁ et R₂ représentent indépendamment un groupe alkyle de 8 à 18 atomes de carbone, benzyle, phényle, 1-naphtyle, ou ledit groupe phényle ou ledit groupe naphtyle étant substitué par un ou deux groupes alkyle de 4 à 12 atomes de carbone,
R₃ représente un atome d'hydrogène,
R₄ représente un atome d'hydrogène ou un groupe méthyle,
R₅ et R₆ représentent indépendamment un atome d'hydrogène ou, lorsque X est un groupe de formule IIIa, un groupe de formule IVa, ou lorsque X est un groupe de formule IIIb, un groupe de formule IVb, et
R₇ représente un atome d'hydrogène.

11. Composé selon la revendication 10, dans lequel
R₁ et R₂ représentent indépendamment un groupe alkyle de 12 à 18 atomes de carbone, benzyle, phényle, 1-naphtyle, ou ledit groupe phényle ou ledit groupe naphtyle étant substitué par un ou deux groupes alkyle de 4 à 8 atomes de carbone,
R₃ représente un atome d'hydrogène,
R₄ représente un atome d'hydrogène,
R₅ représente un atome d'hydrogène ou, lorsque X est un groupe de formule IIIa, un groupe de formule IVa, ou, lorsque X est un groupe de formule IIIb, un groupe de formule IVb,
R₆ représente un atome d'hydrogène ou, lorsque X est un groupe de formule IIIa, un groupe de formule IVa, ou, lorsque X est un groupe de formule IIIb, un groupe de formule IVb, et
R₇ représente un atome d'hydrogène

12. Composé selon la revendication 9 qui est la O-allyl-N,N-dioctadécylhydroxylamine,
la O-allyl-N,N-di(suif hydrogéné)hydroxylamine,
la O-allyl-N,N-dibenzylhydroxylamine,
la O-allyl-N,N-didécylhydroxylamine,
la O,O'-but-2-èn-1,4-diyl-bis (N,N-dibenzylhydroxylamine),
la O,O'-(-2-méthylènepropane-1,3-diyl-bis(N,N-dibenzylhydroxylamine)
la O,O'-but-2-én-1,4-diyl-bis(N,N-diphénylhydroxylamine),
la O-allyl-N,N-diphénylhydroxylamine,
le N-allyl-N,N-dioctadécylamine-N-oxyde,
le N-allyl-N,N-di(suif hydrogéné)amine-N-oxyde,
le N-allyl-N,N-dibenzylamine-N-oxyde,
le N-allyl-N,N-dioctylamine-N-oxyde,
le N,N'-but-2-én-1,4-diyl-bis(N,N-dibenzylamine)-N,N'-dioxyde,
le N,N'-but-2-én-1,4-diyl-bis-(N,N-diphénylamine)-N,N'-dioxyde, ou
le N-allyl-N,N-diphénylamine-N-oxyde.
